# EUROPÄISCHE PATENTSCHRIFT

(11) **EP 1 924 239 B1**
(45) Veröffentlichungstag und Bekanntmachung des Hinweises auf die Patenterteilung: **17.07.2013**
(21) Anmeldenummer: 06791857.3
(22) Anmeldetag: 06.09.2006
(51) Int. Cl.: A61K 8/37, A61Q 19/00, C07C 69/24

(54) **KOSMETISCHE ÖLKÖRPER**
COSMETIC OIL SUBSTANCES
CORPS HUILEUX COSMETIQUES

(30) Priorität: 15.09.2005 EP 05020069; 02.11.2005 DE 102005052173
(43) Veröffentlichungstag der Anmeldung: 28.05.2008
(73) Patentinhaber: Cognis IP Management GmbH, 40589 Düsseldorf (DE)
(72) Erfinder: ANSMANN, Achim, 40699 Erkrath (DE); KAWA, Rolf, 40789 Monheim (DE); ZANDER, Lars, 41569 Rommerskirchen (DE)
(86) Internationale Anmeldenummer: PCT/EP2006/008669
(87) Internationale Veröffentlichungsnummer: WO 2007/031220

(56) Entgegenhaltungen:
- EP-A- 1 149 888
- WO-A-99/06021
- DE-A1- 4 444 137
- DE-C1- 4 341 794
- GB-A- 2 090 612
- "AGNIQUE ME 1270-U" INTERNET CITATION, [Online] 9. Februar 2004 (2004-02-09), XP002374906 Gefunden im Internet: URL:http://www.bsibusiness.com/pdfs/AGNIQU E_ME_1270-U_E.PDF> [gefunden am 2006-03-30]

## Beschreibung

### Gebiet der Erfindung

Die Erfindung befindet sich auf dem Gebiet der kosmetischen Inhaltsstoffe und betrifft Fettsäureestergemische von 2-Ethylhexanol und Fettsäuren, wobei die Summe der C12- und C14- 2-Ethylhexylfettsäureester größer gleich 85%.

### Stand der Technik

Im Bereich kosmetischer Zubereitungen für die Haut- und Haarpflege werden vom Verbraucher eine Vielzahl von Anforderungen gestellt: Abgesehen von reinigenden und pflegenden Effekten, wird Wert auf so unterschiedliche Parameter wie höchstmögliche dermatologische Verträglichkeit, gute rückfettende Eigenschaften, elegantes Erscheinungsbild, optimaler sensorischer Eindruck und Lagerstabilität gelegt.

Zubereitungen, die zur Reinigung und Pflege der menschlichen Haut und der Haare eingesetzt werden, enthalten in der Regel neben einer Reihe von oberflächenaktiven Substanzen, vor allem Ölkörper und Wasser. Als Ölkörper/Emollients werden beispielsweise Kohlenwasserstoffe, Esteröle sowie pflanzliche und tierische Öle/Fette/Wachse eingesetzt. Um die hohen Anforderungen des Marktes bezüglich sensorischer Eigenschaften und optimaler dermatologischer Verträglichkeit zu erfüllen, werden kontinuierlich neue Ölkörper entwickelt und getestet.

Gut spreitende Öle haben in der kosmetischen Industrie einen hohen Stellenwert, da sie ein wesentlicher Faktor für sensorisch leichte Zubereitungen sind. Ein wichtiger Vertreter dieser Gruppe sind die Isopropylester wie Isopropymyristat (IPM) und Isopropylpalmitat (IPP). Der Kosmetikchemiker bemängelt aber gerade bei dieser Produktgruppe sehr häufig eine so genannte "negative Sensorik", das heißt ein deutlich hörbares Knirschen beim Verreiben auf der Haut und eine damit verbundene unangenehme trockene Sensorik. Darüber hinaus haben die Isopropylester den Nachteil, dass sie ein comedogenes Potential besitzen.

Als Ölkörper sind weiterhin Fettsäureester von 2-Ethylhexanol bekannt, wie beispielsweise der Ester von 2-Ethylhexanol mit Stearinsäure (C18), welcher unter der INCI Bezeichnung Ethylhexyl Stearate kommerziell erhältlich sind (z.B. Cetiol® 868 von Fa. Cognis; Crodamol®OS von Croda Inc.; Estol®1514 von Uniquema)

Als Ölkörper sind weiterhin Fettsäureester von 2-Ethylhexanol mit Laurinsäure (C12, Dodecansäure) bekannt, welcher unter der INCI Bezeichnung Ethylhexyl Laurate kommerziell erhältlich sind (z.B. AEC Ethylhexyl Laurate von A & E Connock Ltd; Estol®3613 von Uniquema). Weiterhin bekannt sind Fettsäureester von 2-Ethylhexanol mit Myristinsäure (C14, Tetradecansäure), welche unter der INCI Bezeichnung Ethylhexyl Myristate kommerziell erhältlich sind (z.B. AEC Ethylhexyllaurate von A & E Connock Ltd). Darüber hinaus sind Kokosfettsäureester von 2-Ethylhexylhexanol unter der INCI Bezeichnung Ethylhexyl Cocoate kommerziell (z.B. Crodamol®OC von Croda. Inc) erhältlich. Das Produkt ist eine 2-Ethylhexylfettsäureester mit Fettsäuren des Kokosnussöls. Die Fettsäureverteilen von Kokosnussöl hat folgende typische Zusammensetzung: C 12 (Dodecansäure, Laurinsäure) 45 bis 53 Gew.-%, C 14 (Tetradecansäure, Myristinsäure) 15 bis 21 Gew.-%, C 16 (Palmitinsäure) 7 bis 11 Gew.-%, C 18:1 (Ölsäure) 6 bis 8 Gew.-%, C 18 (Stearinsäure) 2 bis 4 Gew.-%, C 10 (Decansäure) 5 bis 10 Gew.-%, C 8 (Octansäure) 5 bis 10 Gew.-%, C 6 (Hexansäure) unter 1 Gew.-% (Quelle: Ullmanns Encyclopedia of Industrial Chemistry, 2005, Wiley& Sons).

EP 0 732 912 B1 (WO 95/15743) beschreibt Mischungen von Guerbetalkoholen mit 2- Ethylhexylester auf Basis einer Fettsäure mit folgender C-Kette: > 3 Gew.-% C 14, 45 bis 53 Gew.-% C 16, 43 bis 52 Gew.% C 18 und < 2 Gew.% C 18.

Die Aufgabe der Erfindung hat darin bestanden, ein hoch spreitendes Öl zur Verfügung zu stellen, das als Ölkörper selber sowie in kosmetischen und/oder pharmazeutischen Zubereitungen einen glatten Eindruck auf der Haut hinterlässt, geringe Klebrigkeit und hohe Weichheit vermittelt und kein comedogenes Potential aufweist. Überraschenderweise wurde gefunden, dass ein Estergemisch auf Basis 2-Ethylhexanol mit Fettsäuren mit einer definierten Fettsäurenverteilung die erfindungsgemäße Aufgabe löst.

### Beschreibung der Erfindung

Gegenstand der Erfindung sind Fettsäureestergemische von 2-Ethylhexanol und Fettsäuren, wobei die **Summe der C12- und C14- 2-Ethylhexylfettsäureester** größer gleich 85%, bezogen auf die Gesamtsumme der Fettsäureester, beträgt. Bevorzugt ist die Summe der C12- und C14- 2-Ethylhexylfettsäureester größer gleich 90%, insbesondere größer gleich 95%, bezogen auf die Gesamtsumme der Fettsäureester.

Der restliche Anteil besteht üblicherweise aus Fettsäureestern von 2-Ethylhexanol mit Fettsäuren einer C Zahl zwischen C 4 und C 22, insbesondere zwischen C 6 und C 20.

Ein weiterer Gegenstand der vorliegenden Erfindung betrifft ein Fettsäureestergemisch von 2-Ethylhexanol und Fettsäuren, wobei die Summe der 2-Ethylhexylfettsäureester mit einer C-**Kettenlänge von kleiner gleich 10** kleiner gleich 3%, bezogen auf die Gesamtsumme der Fettsäureester, beträgt. Bevorzugt ist die Summe der 2-Ethylhexylfettsäureester mit einer C-Kettenlänge von kleiner gleich 10 kleiner gleich 2 %, insbesondere kleiner gleich 1,5 %, insbesondere kleiner gleich 1 % bezogen auf die Gesamtsumme der Fettsäureester.

Ein weiterer Gegenstand der vorliegenden Erfindung betrifft ein Fettsäureestergemisch von 2-Ethylhexanol und Fettsäuren, wobei die Summe der 2-Ethylhexylfettsäureester mit einer C-**Kettenlänge von größer gleich 16,** kleiner gleich 8% bezogen auf die Gesamtsumme der Fettsäureester, beträgt. Bevorzugt ist die Summe der 2-Ethylhexylfettsäureester mit einer C-Kettenlänge von größer gleich 16, kleiner gleich 4 %, bevorzugt kleiner gleich 3%, insbesondere kleiner gleich 2%, bezogen auf die Gesamtsumme der Fettsäureester.

Keines der bekannten Fettsäureestergemische weist die erfindungsgemäßen 2-Ethyhexylfettsäureester Anteile auf. Überraschenderweise wurde gefunden, dass Gemische mit der erfindungsgemäßen 2-Ethylhexylfettsäureesterverteilung nicht nur hoch spreitende Ölkörper sind, sondern diese gleichzeitig eine hohe Weichheit, hervorragende Pflege sowie dermatologische Verträglichkeit und geringe Klebrigkeit aufweisen. Darüber hinaus haben diese Ölkörper kein comedogenes Potential und lassen sich problemlos in kosmetische Rezepturen einarbeiten.

Eine bevorzugte Ausführungsform der Erfindung betrifft Fettsäureestergemische von 2-Ethylhexanol und Fettsäuren, wobei die **Summe der C12- und C14- 2-Ethylhexylfettsäureester** größer gleich 85%, bezogen auf die Gesamtsumme der Fettsäureester, beträgt und die Summe der 2-Ethylhexylfettsäureester mit einer **C-Kettenlänge von kleiner gleich 10** kleiner gleich 3% beträgt.

Eine bevorzugte Ausführungsform der Erfindung betrifft Fettsäureestergemische von 2-Ethylhexanol und Fettsäuren, wobei die **Summe der C12- und C14- 2-Ethylhexylfettsäureester** größer gleich 85%, bezogen auf die Gesamtsumme der Fettsäureester, beträgt **und** die Summe der 2-Ethylhexylfettsäureester mit einer C-Kettenlänge von **größer gleich 16,** kleiner gleich 8%, bezogen auf die Gesamtsumme der Fettsäureester, beträgt.

Eine bevorzugte Ausführungsform der Erfindung betrifft Fettsäureestergemische von 2-Ethylhexanol und Fettsäuren, wobei die **Summe der C12- und C14- 2-Ethylhexylfettsäureester** größer gleich 85%, bezogen auf die Gesamtsumme der Fettsäureester, beträgt und die Summe der 2-Ethylhexylfettsäureester mit einer **C-Kettenlänge von kleiner gleich 10** kleiner gleich 3% beträgt und die Summe der 2-Ethylhexylfettsäureester mit einer **C-Kettenlänge von größer gleich 16,** kleiner gleich 8%, bezogen auf die Gesamtsumme der Fettsäureester, beträgt.

Bei allen diesen Ausführungsformen beträgt die **Summe der C12- und C14- 2-Ethylhexylfettsäureester** jeweils bevorzugt größer gleich 90%, insbesondere größer gleich 95%, bezogen auf die Gesamtsumme der Fettsäureester.

Bei allen diesen Ausführungsformen beträgt die Summe der 2-Ethylhexylfettsäureester mit einer **C-Kettenlänge von kleiner gleich 10** jeweils bevorzugt kleiner gleich 2 %, insbesondere kleiner gleich 1,5 %, insbesondere kleiner gleich 1 % bezogen auf die Gesamtsumme der Fettsäureester.

Bei allen diesen Ausführungsformen beträgt die Summe der 2-Ethylhexylfettsäureester mit einer **C-Kettenlänge von größer gleich 16** jeweils bevorzugt kleiner gleich 4 %, bevorzugt kleiner gleich 3%, insbesondere kleiner gleich 2%, bezogen auf die Gesamtsumme der Fettsäureester.

Ein weiterer Gegenstand der Erfindung betrifft Fettsäureestergemische von 2-Ethylhexanol und Fettsäuren, wobei die 2-Ethylhexylfettsäureester folgende **C-Kettenverteilung** aufweisen:
(a) C12 größer gleich 60% und (b) C-14 zwischen 15% und 40% bezogen auf die Gesamtsumme der Fettsäureester.

In einer besonders bevorzugten Ausführungsform dieser Erfindung, ist die Summe der C12-Fettsäure 2-Ethylhexylfettsäureester größer gleich 65%, insbesondere größer gleich 70%, bezogen auf die Gesamtsumme der Fettsäureester.

In einer besonders bevorzugten Ausführungsform dieser Erfindung, ist die Summe der C14-Fettsäure 2-Ethylhexylfettsäureester zwischen 20% und 35%, bevorzugt zwischen 25% und 30% bezogen auf die Gesamtsummer der Fettsäureester.

Eine bevorzugte Ausführungsform der Erfindung betrifft Fettsäureestergemische von 2-Ethylhexanol und Fettsäuren, wobei die 2-Ethylhexylfettsäureester folgende **C-Kettenverteilung** aufweisen:
(a) C12 größer gleich 60% und (b) C-14 zwischen 15% und 40% **und** die Summe der 2-Ethylhexylfettsäureester mit einer **C-Kettenlänge von kleiner gleich 10,** kleiner gleich 3%, insbesondere kleiner gleich 2%, bevorzugt kleiner gleich 1%,- jeweils bezogen auf die Gesamtsumme der Fettsäureester beträgt. In einer besonders bevorzugten Ausführungsform dieser Erfindung, ist die Summe der C12-Fettsäure 2-Ethylhexylfettsäureester größer gleich 65%, insbesondere größer gleich 70%, bezogen auf die Gesamtsumme der Fettsäureester. In einer besonders bevorzugten Ausführungsform dieser Erfindung, ist die Summe der C14-Fettsäure 2-Ethylhexylfettsäureester zwischen 20% und 35%, bevorzugt zwischen 25% und 30%.

Eine bevorzugte Ausführungsform der Erfindung betrifft Fettsäureestergemische von 2-Ethylhexanol und Fettsäuren, wobei die 2-Ethylhexylfettsäureester folgende **C-Kettenverteilung** aufweisen:
(a) C12 größer gleich 60% und (b) C-14 zwischen 15% und 40% **und** die Summe der 2-Ethylhexylfettsäureester mit einer **C-Kettenlänge von größer gleich 16,** kleiner gleich 8%, insbesondere in kleiner gleich 4%, bevorzugt kleiner gleich 3%, insbesondere kleiner gleich 2%, bevorzugt kleiner gleich 1%,- jeweils bezogen auf die Gesamtsumme der Fettsäureester, beträgt. In einer besonders bevorzugten Ausführungsform dieser Erfindung, ist die Summe der C12-Fettsäure 2-Ethylhexylfettsäureester größer gleich 65%, insbesondere größer gleich 70%, bezogen auf die Gesamtsumme der Fettsäureester. In einer besonders bevorzugten Ausführungsform dieser Erfindung, ist die Summe der C14-Fettsäure 2-Ethylhexylfettsäureester zwischen 20% und 35%, bevorzugt zwischen 25% und 30%.

Eine besonders bevorzugte Ausführungsform der Erfindung betrifft Fettsäureestergemische von 2-Ethylhexanol und Fettsäuren, wobei die 2-Ethylhexylfettsäureester folgende **C-Kettenverteilung** aufweisen:
(a) C12 größer gleich 60% und (b) C-14 zwischen 15% und 40% **und** die Summe der 2-Ethylhexylfettsäureester mit einer **C-Kettenlänge von kleiner gleich 10,** kleiner gleich 3%, insbesondere kleiner gleich 2%, bevorzugt kleiner gleich 1%, beträgt **und** die Summe der 2-Ethylhexylfettsäureester mit einer **C-Kettenlänge von größer gleich 16,** kleiner gleich 8%, insbesondere in kleiner gleich 4%, bevorzugt kleiner gleich 3%, insbesondere kleiner gleich 2%, bevorzugt kleiner gleich 1 %,- jeweils bezogen auf die Gesamtsumme der Fettsäureester, beträgt. In einer besonders bevorzugten Ausführungsform dieser Erfindung, ist die Summe der C12-Fettsäure 2-Ethylhexylfettsäureester größer gleich 65%, insbesondere größer gleich 70%, bezogen auf die Gesamtsumme der Fettsäureester. In einer besonders bevorzugten Ausführungsform dieser Erfindung, ist die Summe der C14-Fettsäure 2-Ethylhexylfettsäureester zwischen 20% und 35%, bevorzugt zwischen 25% und 30%.

### Herstellung

Die Herstellung der erfindungsgemäßen Fettsäureestergemische erfolgt nach dem Fachmann bekannten Verfahren. Die Fettsäureestergemische können beispielsweise durch Veresterung von 2-Ethylhexanol mit Fettsäuregemischen hergestellt werden, wobei die Fettsäuregemische die erfindungsgemäße Fettsäureverteilung aufweisen, welche sich dann im Fettsäure-2-Ethylhexylestergemisch wieder findet. Ebenso kann die Herstellung erfolgen durch Umesterung von 2-Ethylhexanol mit einem Fettsäuremethylestergemisch, wobei das zur Herstellung eingesetzte Fettsäuremethylestergemisch die erfindungsgemäße Fettsäureverteilung aufweist. Weiterhin können die erfindungsgemäßen Fettsäureestergemische durch Mischen der entsprechenden einzelnen Fettsäure-2-Ethylhexylester erhalten werden.

Der Geruch der durch Veresterung oder Umesterung erhaltenen Produkte kann je nach Bedarf durch Desodorieren verbessert werden, ebenso kann die Farbe gegebenenfalls durch Behandlung mit dem Fachmann bekannten Methoden verbessert werden.

Übliche Fettsäuregemische bzw. Fettsäuremethylestergemische, welche sich für die Herstellung der erfindungsgemäßen Fettsäure-2-Ethylhexylestergemische eignen, weisen beispielsweise folgende C-Kettenverteilung auf: Summe der C6 bis C10 Fettsäure ist kleiner gleich 3%, die Summe der C 16 Fettsäuren beträgt 60 bis 80 %, C14 Fettsäure 15 bis 40 %, C 16 kleiner gleich 4%, Summe der Fettsäuren mit einer C-Kette größer 16 kleiner gleich 0,5 %.

Fettsäuregemische, welche sich zur Herstellung der erfindungsgemäßen Estergemische eignen sind beispielsweise unter dem Handelsnamen Edenor®C12 70 (Fa. Cognis) erhältlich und weisen folgende Fettsäureverteilung bezogen die Gesamtsumme der Fettsäuren auf: Summe der Fettsäuren mit einer C-Zahl ≤ 10 = 2 %, bevorzugt ≤1 %; C12-Fettsäuren zwischen 65 und 77 %, bevorzugt 68 %; C14 Fettsäure zwischen 19 und 34 %, bevorzugt 28 %; C 16 Fettsäure ≤ 4 %, insbesondere ≤ 3%.

Unter dem in Begriff "C X Fettsäuren" sind erfindungsgemäß alle Carbonsäuren zusammengefasst, welche eine C-Zahl von "X" aufweisen. So umfasst beispielsweise der Begriff "C12 Fettsäuren" alle Carbonsäuren mit einer C-Zahl von 12. Gleiches gilt für den entsprechend verwendeten Begriff "C-Y Fettsäure-2-Ethylhexylester".

Umfasst sind sowohl aliphatische, aromatische, gesättigte, einfach sowie mehrfach ungesättigte, lineare und verzweigte Fettsäuren. In einer bevorzugten Ausführungsform der Erfindung werden hauptsächlich (d.h. in der Regeln über 90% der jeweiligen Fettsäure) aliphatische, linearen Carbonsäuren der angegebenen C-Zahl eingesetzt, wie beispielsweise Laurinsäure als C12 Fettsäure und Myristinsäure als C14 Fettsäure.

### Kosmetische und/oder pharmazeutische Zubereitungen

Die erfindungsgemäßen Fettsäure-2-Ethylhexylestergemische erlauben die Herstellung stabiler kosmetischer und pharmazeutischer Emulsionen.

Ein weiterer Gegenstand der vorliegenden Erfindung betrifft daher die Verwendung der erfindungsgemäßen Fettsäure-2-Ethylhexylestergemische in kosmetischen und/oder pharmazeutischen Zubereitungen, insbesondere als Ölkörper. Die erfindungsgemäßen Fettsäure-2-Ethylhexylestergemische können dabei, je nach Zubereitung, sowohl als alleinige Ölkörper als auch in Kombination mit weiteren Ölkörpern eingesetzt werden.

Die erfindungsgemäßen Fettsäure-2-Ethylhexylestergemische können in kosmetischen und/oder pharmazeutischen Zubereitungen in Konzentrationen von 1 bis 90% eingesetzt werden. Der bevorzugte Einsatzbereich liegt zwischen 1 bis 50 %, insbesondere 2% und 20%, bezogen auf das Gesamtgewicht der kosmetischen und/oder pharmazeutischen Zubereitung. Einsatzgebiete sind beispielsweise kosmetische und/oder pharmazeutische O/W oder W/O Pflegeemulsionen, Sonnenschutzformulierung, AP/Deo Konzepte, Formulierungen für die dekorative Kosmetik, ölige Pflegezubereitungen, Tränkflüssigkeiten für Substrate, wie beispielsweise Papier- und Vliessprodukte. Exemplarisch seien genannt Wet Wipes, Taschentücher, Windeln oder Hygieneprodukte.

Die erfindungsgemäßen Fettsäure-2-Ethyhexylestergemische eignen sich inbesondere für eine sprühbare Anwendung und/oder als Pflegeemulsion für Tissues, Papiere, Wipes, Schwämme (z.B. Polyurethanschwämme), Pflaster im Bereich Baby-Hygiene, Baby-Pflege, Hautpflege, Sonnenschutz, After-SunTreatment, Insektrepellent, Reinigung, Gesichtsreinigung und AP/Deo - Anwendung geeignet sind. Durch den Einsatz der erfindungsgemäßen Fettsäure-2-Ethyhexylestergemische wird das sensorische Verhalten bei Applikation positiv beeinflusst.

Die kosmetischen und/oder pharmazeutischen Zubereitungen können Formulierungen zur Körperpflege sein, z. B. eine Körpermilch, Cremes, Lotionen, sprühbare Emulsionen, Produkte zur Eliminierung des Körpergeruchs etc. Die Fettsäure-2-Ethylhexylestergemische lassen sich auch in tensidhaltigen Formulierungen wie z. B. Schaum- und Duschbädem, Haarshampoos und Pflegespülungen einsetzen. Je nach Applikationszweck enthalten die kosmetischen Formulierungen eine Reihe weiterer Hilfs- und Zusatzstoffe, wie beispielsweise Tenside, weitere Ölkörper, Emulgatoren, Pedglanzwachse, Konsistenzgeber, Verdickungsmittel, Überfettungsmittel, Stabilisatoren, Polymere, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosinaseinhibitoren (Depigmentierungsmittel), Hydrotrope, Solubilisatoren, Konservierungsmittel, Parfümöle, Farbstoffe etc., die nachstehend exemplarisch aufgelistet sind.

Als oberflächenaktive Stoffe können anionische, nichtionische, kationische und/oder amphotere bzw. zwitterionische **Tenside** enthalten sein. In tensidhaltigen kosmetischen Zubereitungen, wie beispielsweise Duschgelen, Schaumbädern, Shampoos etc. ist vorzugsweise wenigstens ein anionisches Tensid enthalten. Der Anteil der Tenside liegt hier üblicherweise bei etwa 1 bis 30, vorzugsweise 5 bis 25 und insbesondere 10 bis 20 Gew.-%.

Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymisch-ethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

Körperpflegemittel, wie Cremes, Lotionen und Milchen, enthalten üblicherweise eine Reihe **weiterer Ölkörper** und Emollients, die dazu beitragen, die sensorischen Eigenschaften weiter zu optimieren. Die Ölkörper können, ja nach Art der Formulierung in einer Gesamtmenge von 1 bis 90 Gew.-%, insbesondere in einer Gesamtmenge von 1 - 50 Gew.-%, vorzugsweise 5 - 25 Gew.-% und insbesondere 5 - 15 Gew.-% enthalten sein. Als weitere Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z. B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z. B. Dica-prylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Dicaprylyl Ether (Cetiol® OE), Ringöffnungsprodukte von epoxidierten Fettsäureestern mit Polyolen.

**Fette und Wachse** werden den Körperpflegeprodukten als Pflegestoffe zugesetzt und auch, um die Konsistenz der Kosmetika zu erhöhen. Typische Beispiele für Fette sind Glyceride, d. h. feste pflanzliche oder tierische Produkte, die im Wesentlichen aus gemischten Glycerinestern höherer Fettsäuren bestehen. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, -palmitat oder -stearat kommen hierfür in Frage. Als Wachse kommen u. a. natürliche Wachse, wie z. B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z. B. Montanesterwachse, Sasolwachse, hydrierte Jojobawachse sowie synthetische Wachse, wie z. B. Polyalkylenwachse und Polyethylenglycolwachse in Frage. Neben den Fetten kommen als Zusatzstoffe auch fettähnliche Substanzen, wie Lecithine und Phospholipide in Frage. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Als Verdickungsmittel eignen sich beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, Polyvinylalkohol, Polyvinylpyrrolidon und Bentonite wie z. B. Bentone^{®} Gel VS-5PC (Rheox).

Erfindungsgemäß sind als **UV-Lichtschutzfaktoren** bei Raumtemperatur flüssige oder kristalline organische Substanzen (Lichtschutzfilter) geeignet, die in der Lage sind, ultraviolette Strahlen zu absorbieren und die aufgenommene Energie in Form längerwelliger Strahlung, z.B. Wärme wieder abzugeben. UV-B-Filter können öllöslich oder wasserlöslich sein. Als öllösliche Substanzen sind z.B. zu nennen:
➢ 3-Benzylidencampher bzw. 3-Benzylidennorcampher und dessen Derivate, z.B. 3-(4-Methylbenzyliden)campher wie in der EP 0693471 B1 beschrieben;
➢ 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoe-säureamylester;
➢ Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-iso-propylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexyl-ester;
➢ Triazinderivate, wie z.B. 2,4,6-Trianilino-(p-carbo-2'-ethyl-1'-hexyloxy)-1,3,5-triazin und Octyl Triazon, wie in der EP 0818450 A1 beschrieben oder Dioctyl Butamido Triazone (Uvasorb® HEB);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der EP 0694521 B1 beschrieben.

Als wasserlösliche Substanzen kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ Sulfonsäurederivate von Benzophenonen, vorzugsweise 2-Hydroxy-4-methoxybenzo-phenon-5-sulfonsäure und ihre Salze;
➢ Sulfonsäurederivate des 3-Benzylidencamphers, wie z.B. 4-(2-Oxo-3-bornylidenmethyl)-benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der DE 19712033 A1 (BASF) sowie Benzoic Acid, 2-[4-(Diethylamino)-2-Hydroxybenzol]-, Hexyl Ester (Uvinul® A plus). Die UV-A und UV-B-Filter können selbstverständlich auch in Mischungen eingesetzt werden. Besonders günstige Kombinationen bestehen aus den Derivaten des Benzoylmethans,, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Besonders geeignete Breitspektrum-Sonnenfilter sind 2,2'-Methylenebis [4-(1,1,3,3-Tetramethylbutyl)-6-(2H-Benzotriazol-2-yl)Phenol] (Tinosorb M) und Phenol, 2,2'-[6-(4-Methoxyphenyl)-1,3,5-Triazine-2,4-Diyl]Bis[5-[(2-Ethylhexyl)Oxy]- (Tinosorb M).

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und auch für die dekorative Kosmetik verwendet. Die Partikel sollten einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 543 (1996) sowie Parf.Kosm. 3, 11 (1999) zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Unter **biogenen Wirkstoffen** sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

**Desodorierende Wirkstoffe** wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend eignen sich als deosodorierende Wirkstoffe u. a. keimhemmende Mittel, Enzyminhibitoren, Geruchsabsorber oder Geruchsüberdecker.

Als **Insekten-Repellentien** kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent^{®} 3535 von der Merck KGaA vertrieben wird, sowie Butylacetylaminopropionate in Frage.

Als **Selbstbräuner** eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Als **Konservierungsmittel** eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Als **Parfümöle** seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzem, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

Als **Perlglanzwachse,** insbesondere für den Einsatz in tensidischen Formulierungen, kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Stabilisatoren** können Metallsalze von Fettsäuren, wie z. B. Magnesium-, Aluminium- und/oder Zinkstearat bzw. -ricinoleat eingesetzt werden.

Zur Verbesserung des Fließverhaltens können ferner **Hydrotrope,** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

### Beispiele

### Erfindungsgemäßes Beispiel A- Herstellung

1300 g 2-Ethylhexanol und 1750 g eines Fettsäuregemisch (Edenor®C12 70%, Fa. Cognis) mit folgender Fettsäureverteilung C10 </= 2%, C12 65-77 %, C 14 19-34%, C16 </= 4% wurden in Gegenwart eines Farbinhibitors (0,41 g 50%ige unterphosphorige Säure) und Katalysators (0,25 g Zinn-(II)-oxid) bei Normaldruck vorgelegt. Das Gemisch wurde unter N₂ Atmosphäre auf 245 °C erhitzt. Sobald die Säurezahl von 10 unterschritten war, wurde ein Vakuum von 300 mbar angelegt, sobald die Säurezahl von 1,5 unterschritten wurde, wurde der Phasenabscheider umfahren, sobald die Säurezahl von 1 unterschritten wurde, wurde das überschüssige 2-Ethylhexanol abdestilliert (bei ca. 5mbar).

Im Raffinationsbehälter wurde der Katalysator mit Phosphorsäure (75% der Zinn-(II)-oxid Menge) und 200 l Wasser gefällt. Die Trocknung erfolgte bei 85°C und etwa 5 bis 50 mbar.

### Vergleichsbeispiel B

Als Vergleichsbeispiel dient das kommerziell erhältiche Produkt Crodamol®OC (Fa. Croda). Die folgende Tabelle zeigt die C-Kettenverteilung der erfindungsgemäßen Beispiele A und C sowie des Vergleichsbeispiels B (= Ethylhexylfettsäureester, INCI: Ethylhexylcocoate, Fa. Croda).

| | Erfindungsgemäßes Beispiel A | Erfindungsgemäßes Beispiel C | Vergleichsbeispiel B Crodamol®OC, (Fa. Croda) |
|---|---|---|---|
| C6 [%] | Summe der Fettsäuren mit C ≤ C8 < 0,5% | 0 | 0,1 |
| C8 [%] | | 0 | 4,2 |
| C10 [%] | < 1 % | 0,1 | 6,9 |
| C12 [%] | 68 | 73,4 | 47,6 |
| C14 [%] | 28 | 25,3 | 20,5 |
| C16 [%] | 2 | Summe der Fettsäuren mit C ≥ C 16 = 1,2 | 12,6 |
| C18 [%] | Summe der Fettsäuren mit C > C18 ist < 0,5 % | | 1,4 |
| C18:1 [%] | | | 6,3 |
| C18:2 [%] | | | 0,5 |

### Beispiel 1 Sensorische Bewertung

Die sensorische Bewertung des erfindungsgemäßen Beispiels A erfolgte zum Vergleichsbeispiel B sowie zu Isopropylpalmitat, einem typischen kosmetischen Ölkörper.

Ein Panel bestehend aus 12 Experten führte die sensorische Bewertung durch. Abgefragt wurden die folgenden 5 Kriterien bezogen auf das Endgefühl auf der Haut:
1 - spreitend, 2 - negative Sensorik, 3 - klebrig, 4 - Weichheit, 5- Pflegegefühl.

Die Bewertung dieser Kriterien erfolgte mit Noten von 1 (wenig) bis 7 (viel)

| Kriterien | Isopropylpalmitat | Erfindungsgemäßes Beispiel A | Vergleichsbeispiel B Crodamol®OC (Fa. Croda) |
|---|---|---|---|
| spreitend | 7 | 7 | 3 |
| negative Sensorik | 7 | 1 | 1 |
| Klebrigkeit | 2 | 1 | 3 |
| Weichheit | 2 | 7 | 3 |
| Pflegegefühl | 2 | 7 | 3 |

Das erfindungsgemäße Beispiel A zeigt gegenüber dem Stand der Technik hohe Weichheit und hohes Pflegegefühl bei gleichzeitig sehr guten spreitenden Eigenschaften sowie vorteilhaften sensorischen Eigenschaften (u.a. geringe Klebirgkeit).

### Beispiel 2 Sensorische Beurteilung in kosmetischen Formulierungen

Für die Bewertung der sensorischen Parameter wurden die folgenden kosmetischen Emulsionen hergestellt:

| | INCI | Rezeptur 1 (erfindungsgemäß) | Rezeptur 2 | Rezeptur 3 |
|---|---|---|---|---|
| Emulgade®SE-PF (Fa. Cognis) | Glyceryl Stearate (and) Ceteareth-20 (and) Ceteareth-12 (and) Cetearyl Alcohol (and) Cetyl Palmitate | 6,0 | 6,0 | 6,0 |
| Ethylhexylester nach Herstellbeispiel A | | 16,0 | - | - |
| Vergleichsbeispiel B Crodamol® OC (Fa. Croda) | Ethylhexylcocoate | - | 16,0 | - |
| Isopropylpalmitate | | - | - | 16,0 |
| Cosmedia® SP (Fa. Cognis) | Sodium polyacrylate | 0,2 | 0,2 | 0,2 |
| Glycerin | | 3,0 | 3,0 | 3,0 |
| Wasser, Konservierungsmittel | | 74,8 | 74,8 | 74,8 |

Alle Angaben sind in Gewichtsprozent. Emulgade® SE-PF und die jeweilige Ölkomponente wurden bei 75°C geschmolzen. Cosmedia®SP wurde homogen eingerührt, Wasser und Glycerin von ebenfalls 75°C wurden zu der Ölphase gegeben und homogen verrührt. Im Anschluss wurde abgekühlt. Bei ca. 55°C erfolgte ein Homogenisierschritt mit einem geeigneten Rotor / Stator System. Die Zugabe des Konservierungsmittels erfolgte in Abhängigkeit von der Temperaturempfindlichkeit entweder bei 75°C oder bei 40°C.

Die sensorische Bewertung erfolgte wie oben angegeben.

| Kriterien | Rezeptur 1 (erfindungsgemäß) | Rezeptur 2 (Ethylhexyl Cocoate, Crodamol® OC) | Rezeptur 3 (Isopropylpalmitate) |
|---|---|---|---|
| Spreitend | 6 | 3 | 5 |
| negative Sensorik | 1 | 1 | 3 |
| Klebrigkeit | 1 | 3 | 1 |
| Weichheit | 7 | 3 | 3 |

### Kosmetische Zubereitungen : Formulierungen für Spray - und Wipe - Anwendungen sowie für AP / Deo Konzepte

Die Rezepturen 1 bis 26 beschreiben stabile Formulierungen auf Basis der erfindungsgemäßen Ölkomponente, insbesondere des Herstellungsbeispiels A, die besonders für eine sprühbare Anwendung und/oder als Pflegeemulsion für Tissues, Papiere, Wipes, Schwämme (z.B. Polyurethanschwämme), Pflaster im Bereich Baby-Hygiene, Baby-Pflege, Hautpflege, Sonnenschutz, After-SunTreatment, Insektrepellent, Reinigung, Gesichtsreinigung und AP/Deo - Anwendung geeignet sind. Durch den Einsatz der erfindungsgemäßen Ölkomponente wird das sensorische Verhalten bei Applikation positiv beeinflusst. Die Mengenangaben beziehen sich jeweils auf Gew.-% der handelsüblichen Substanzen in der Gesamtzusammensetzung.

**Tabelle 1: Rezepturen 1 bis 13**

| **Komponenten INCI (Handels-name)** | **1** | **2** | **3** | **4** | **5** | **6** | **7** | **8** | **9** | **10** | **11** | **12** | **13** |
|---|---|---|---|---|---|---|---|---|---|---|---|---|---|
| Emulgade^{®} SE | | | | | | | | | | | | 10.7 | 5.1 |
| Eumulgin^{®} B2 | | | | | | | | | | | | 5.8 | 3.4 |
| Emulgade^{®} PL 68/50 | 1 | | 1 | 1 | | 2 | 2 | | 2 | | 2 | | |
| Eumulgin^{®} VL 75 | | 1 | | | 1 | | | 3 | | 2.5 | | | |
| Lanette^{®} E | 1 | 1 | 1 | | 1 | | | | 1 | 1 | | | |
| **Erfindungsgemäße Ölkomponente** | **5** | **4** | **8** | **3** | **5** | **8** | **4** | **2** | **4** | **3** | **5** | **10** | **2** |
| Cetiol^{®} CC | 5 | 5 | 5 | | | | | 4 | | 5 | 3 | 4 | |
| Myritol^{®} 331 | 3 | 4 | | 4 | 4 | | | | 5 | | | 3 | 3 |
| Cetiol^{®} OE | | | | | 5 | | 3 | | 2 | | | | |
| Cetiol^{®} B | | | | 4 | | | | 4 | | 4 | | | |
| Cosmedia^{®} DC | 1 | 1 | 1 | 1 | 1 | 1.5 | 1.5 | 2 | 3 | 2 | 1.5 | 2 | 2 |
| Insect Repelient^{®} 3535 | | | | | | | | | | | 5 | 5 | |
| Copherol^{®} F1300 C | 1 | 1 | 1 | 1 | 1 | 1 | 1 | 1 | 1 | 1 | 1 | 1 | 1 |
| Zink oxide NDM | 5 | 5 | 5 | 5 | 5 | | 2 | | 5 | 3 | | | |
| Eusolex^{®} T 2000 | | | | 5 | 5 | | 2 | 3 | 5 | 2 | | | |
| Neo Heliopan^{®} AV | 7.5 | 7.5 | 7.5 | | | | 3 | 1 | 3 | 5 | | 5 | 5 |
| Neo Heliopan^{®} AV | 9 | 9 | 9 | | | 2 | 1 | | | | 2 | | 1.5 |
| Parsot^{®} 1789 | | | | | | 2 | 2 | | | 1 | 2 | 2 | |
| Neo Heliopan^{®} MBC | | | | | | | 2 | | | | | | 2 |
| Uvinul^{®} T 150 | | | | | | 1 | 1 | 2 | | | 1 | | |
| Uvasorb^{®} HEB | | | | | | 1 | 1 | 2 | | | 1 | 2 | |
| Neo Heliopan^{®} Hydro - Na-Salz, 15% wäßrige Lsg. | | | | | | | | | | | | | 13.3 |
| Glycerin | 3 | 3 | 3 | 3 | 3 | 3 | 3 | 3 | 3 | 3 | 3 | 5 | 5 |
| Veegum^{®} plus | 0.75 | 0.75 | | 0.5 | 0.5 | | 0.5 | | | 0.35 | | | |
| Keltrol T | 0.25 | 0.25 | | 0.5 | 0.5 | | 0.5 | | | 0.35 | | | |
| Cosmedia^{®} SP | | | 0.1 | 0.1 | | 0.1 | 0.2 | | | | 0.1 | | |
| Permulen^{®} TR-2 Polymer | | | | | | | | 0.2 | 0.1 | | | | |
| Wasser, Parfum, Konservierungsmittel | q.s. | q.s. | q.s. | q.s. | q.s. | q.s. | q.s. | q.s. | q.s. | q.s. | q.s. | q.s. | q.s. |

**Tabelle 2 Rezepturen 14 bis 26**

| **Komponenten INCI (Handelsname)** | **14** | **15** | **16** | **17** | **18** | **19** | **20** | **21** | **22** | **23** | **24** | **25** | **26** |
|---|---|---|---|---|---|---|---|---|---|---|---|---|---|
| Emulgade^{®} SE | 37 | 3.7 | | | | | | | | | | 4.9 | 4.1 |
| Eumulgin^{®}B1 | 1.3 | 1.3 | 1.3 | | | | | | | | | | |
| Eumulgin^{®}B2 | | | | | | | | | | | | 1.1 | 0.9 |
| Emulgade^{®} PL 68/50 | | | 5 | 1 | 1 | 1 | 1 | 3 | | | | | |
| Eumulgin^{®} VL 75 | | | | | | | | | 3 | 5 | 5 | | |
| Lanette^{®} E | | | | 0.25 | 0.25 | 0.25 | 0.25 | 25 | | | | | |
| Amphisol K | | | 0.5 | | | | | | | | | | |
| **erfindungsgemäße Ölkomponente** | 4 | 5 | 6 | 8 | 5 | 8 | 8 | 10 | 7 | 4 | 10 | 5 | 5 |
| Cetiol^{®} CC | 5 | | 5 | | | | | | 2.5 | 4 | 4 | 5 | 5 |
| Cetiol^{®} LC | | | | 1 | 1 | 1 | 1 | 1 | | | | | |
| Myritol^{®} 312 | | | | 1 | 1 | 1 | 1 | 1 | | | | | |
| Myritol^{®} 331 | | | | | | | | | | 4 | 4 | | |
| Cetiol^{®} SN | 3 | 3 | 3.5 | | | | | | | | | | |
| Eutanol^{®} G | | | | | | | | | 3.5 | 2 | 2 | | |
| Eutanol^{®} G16 | | | | 1 | 1 | 1 | 1 | 1 | | | | | |
| Cegesoft^{®} PS6 | | 1.5 | 15 | | | | | | | | | | |
| Cegesoft^{®} PFO | 1.5 | | | | | | | | | | | | |
| Silikonöl Wacker AK^{®} 350 | | | | 0.5 | 0.5 | 0.5 | 0.5 | 0.5 | | | | | |
| Cosmedia^{®} DC | 1 | | 1.5 | | | | | 1.5 | | 2,5 | 2,5 | | 0.5 |
| Hvdagen^{®} C.A.T | | | | | | | | | | | | 1.5 | |
| Copherol^{®} F 1300 C | | | | | | | | | 0.5 | 0.5 | 0.5 | | |
| Copherol^{®} 1250 C | 0.5 | 0.5 | | | | | | | | | | | |
| Ethanol | | | | | | | | | | | 5 | | |
| Locron^{®} L | | | | | | | | | | | | | 40 |
| Hydagen^{®} DCMF | | | | | | | | | | | | 0.1 | |
| Glycolic Acid | | | | | | | | | | | | 0.04 | |
| Glycerin | 2 | 2 | 2 | 3 | 3 | 3 | 3 | 3 | 2 | 2 | 2 | 3 | 2 |
| Potassium Hydroxyde, 20% wäßrige Lsg. | | | | | | 0.3 | 0.2 | 0.1 | 0.4 | 03 | 0.5 | | |
| Hispagel^{®} 50 | | | | | | | | | | 10 | | | |
| Carbomer | | | | | | | 0.1 | | 0.2 | | 02 | | |
| Cosmedia^{®} SP | | | | | 015 | | | | | | | | |
| Permulen^{®} TR-2 Polymer | | | | | | 0.15 | | 0.05 | | | | | |
| Wasser, Parfum, Konservierungsmittel | q.s. | q.s. | q.s. | q.s. | q.s. | q.s. | q.s. | q.s. | q.s. | q.s. | q.s. | q.s. | |

**Tabelle 3: Rezepturen 27 bis 33 (Formulierungen für AP/Deo)**

| **Komponenten INCI (Handelsname)** | **27** | **28** | **29** | **30** | **31** | **32** | **33** |
|---|---|---|---|---|---|---|---|
| Emulgade^{®} SE-PF | 6 | | 4,5 | | | 6 | |
| Ceteareth-12 (Eumulgin^{®}B1) | | | | | | | |
| Ceteareth-20 (Eumulgin^{®}B2) | | | 1 | | | | |
| Emulgade^{®} CM | | | | | 20 | | |
| Lameform^{®} TGI | | 3 | | | | | |
| Novata^{®} AB | | | | | | | 4 |
| Lanette^{®} 18 | | | | 14,7 | | | |
| Cutina^{®} HR | | | | 3,7 | | | 6,5 |
| Dehymuls^{®} PGPH | | 1 | | | | | |
| Lanette^{®} E | 0,3 | | | | | 0,3 | |
| Lanette^{®} 22 | 2 | | | | | 4 | |
| **Erfindungsgemäße Ölkomponente** | 4 | 4 | 5 | 5 | 4 | 4 | 15 |
| Cetiol^{®} CC | | 3 | | | | | |
| Cetiol^{®} OE | 2 | | | 4 | | 3 | 9 |
| Myritol^{®} 331 | | | | | | | |
| Cetiol^{®} S | | | 5 | 14,7 | | | 20 |
| Dow Corning^{®} 246 Fluid | 3 | 5 | | 34 | | 2 | 14 |
| SFE^{®} 839 (GE Bayer) | | 3 | | | | | |
| Silikonöl Wacker AK^{®} 350 | 1 | | | | | | |
| Cosmedia^{®} DC | 1 | 1 | 1 | 1 | 1 | 1 | 1 |
| Hydagen^{®} C.A.T | | | 2 | | | | |
| Eumulgin^{®} HRE 40 | | | | | 1 | | |
| Copherol^{®} 1250 C | | | | 1 | | | |
| Rezal^{®} 36 | 30 | 40 | | 22,9 | | 30 | 25 |
| Locron^{®} L | | | 10 | | | | |
| Hydagen^{®} DCMF | 0,05 | | | | | | |
| Glycolic Acid | 0,02 | | | | | | |
| Glycerin | | 5 | 5 | | | | |
| propylene Carbonate | | | | | | | 0,5 |
| Bentone^{®} 18 | | | | | | | 1 |
| Talkum | | | | | | 5 | 5 |
| MgSO4x 7H2O | | 1 | | | | | |
| Wasser Phase II | 46,7 | | 35 | | | | |
| Wasser, Parfum, Konservierungsmittel | q.s. | q.s. | q.s. | q.s. | q.s. | q.s. | q.s. |

| | | | | | | | |
|---|---|---|---|---|---|---|---|
| 27 - Antiperspirant / Deo Creme 28 - Antiperspirant Creme (W/O) 29 - Antiperspirant / Deo Spray 30 - Antiperspirant Stift mit Vitamin E 31 - Deodorant Wipe - Formulierung 32 - Antiperspirant Creme 33 - Antiperspirant Creme «Soft Solid » | | | | | | | |

In der Tabelle 4 werden Sonnenschutzformulierungen vom Typ O/W beschrieben, in der Tabelle 5 werden Pflegeemulsionen beschrieben. Durch den Einsatz der erfindungsgemäßen Ölkomponente wird das sensorische Verhalten bei Applikation positiv beeinflusst Die Mengenangaben beziehen sich jeweils auf Gew.-% der handelsüblichen Substanzen in der Gesamtzusammensetzung.

**Tabelle 4: O/W-Sonnenschutzemulsionen**

| **Komponente** | **1** | **2** | **3** | **4** | **5** | **6** | **7** | **8** | **9** | **10** | **11** |
|---|---|---|---|---|---|---|---|---|---|---|---|
| **L = Lotion, C = Creme, S = Spray** | L | C | L | C | L | C | S | C | C | L | L |
| Eumulgin^{®} VL 75 | 2 | | | | 3 | | | | 1 | | |
| Eumulgin^{®} B2 | | | | 2 | | | | | | 1 | |
| Tween^{®} 60 | | | | | | | | | | 1 | |
| Cutina^{®} E 24 | | | | 0.5 | | | | | | | |
| Hostaphat^{®} KL 340 N | | | | | | | | | | | 0.5 |
| Eumulgin^{®} SG | | | 0,5 | | | 0,5 | | 0,3 | 0,1 | | |
| Lanette^{®} E | | | | | | | 0.1 | | 0.5 | | |
| Amphisol^{®} K | 05 | | | | | | 1 | | | | |
| Natriumstearat | | | | | 1 | | | | | | |
| Emulgade^{®} PL 68/50 | | 2 | 1 | | | 3 | | | | | |
| Tego^{®} Care 450 | | 2 | | | | | | | 2 | | |
| Cutina^{®} MD | | | | 2 | 1 | 3 | | | | | 1 |
| Lanette^{®} 14 | | 1 | | | | | | | | | |
| Lanette^{®} O | | | | 2 | | | | 2 | 1 | 1 | |
| Cutina^{®} PES | 1 | 1 | | 2 | | | | | | 1 | |
| Allianz^{®} OPT | 1 | | | 1 | 1 | | | 2 | | | 2 |
| Cosmedia^{®} DC | | 1.5 | 2 | | | 1.5 | 2 | | 1.5 | 1.5 | |
| Emery^{®} 1780 | | | | 1 | 1 | | | | | | |
| Lanolin wasserfrei USP | | | | | | 1 | 1 | | | | |
| **erfindungsgemäße Ölkomponente** | **6** | **2** | **4** | **7** | **3** | **7** | **6** | **6** | **4** | **4** | **5** |
| Myritol^{®} PC | | | | | | | | | 5 | | |
| Myritol^{®} 331 | 6 | | 4 | | | 5 | 8 | | | 10 | 8 |
| Finsolv^{®} TN | | | | | 5 | | | 3 | 3 | | |
| Cetiol^{®} CC | 6 | | 6 | | | 5 | 5 | | | | |
| Cetiol^{®} OE | | | | | 2 | | | | | | 2 |
| Dow Corning DC^{®} 244 | | 2 | | | 1 | | | | | | |
| Dow Corning DC^{®} 2502 | | 1 | | | 1 | | | 3 | | | |
| Ceraphyl^{®} 45 | | | | | | | | | | 2 | 2 |
| Silikonöl Wacker AK^{®} 350 | | | | | 1 | | | | | | |
| Cetiol^{®} 868 | | 2 | | | | | | | | | |
| Cetiol^{®} J 600 | | 2 | | | | | | | | | |
| Mineralöl | | | | 5 | | | | | | | |
| Cetiol^{®} B | 4 | | 4 | | | | | 4 | | | |
| Eutanol^{®} G | | 3 | | | | 3 | | | | | |
| Eutanol^{®} G 16 S | 3 | | | | | | | | | | |
| Cetiol^{®} PGL | | | | | | | | | 2 | | |
| Photonyl^{®} LS | | | | | | | | | | 2 | |
| Panthenol | 1 | | | | | | | | | | |
| Bisabolol | 0,2 | | | | | | | | | | |
| Tocopherol / Tocopherylacetat | 1 | | | | | | | | | | |
| Neo Heliopan^{®} Hydro (Na-Salz) | | | | | | | | | | 3 | |
| Eusolex^{®} OCR | 6 | | 9 | | 5 | 7 | 9 | | 4 | | 7 |
| Neo Heliopan^{®} AP (Na-Salz) | | | | 0.5 | | 1 | | | | | |
| Neo Heliopan^{®} BB | | | | | | | | 1 | 1 | | 1 |
| Neo Heliopan^{®} MBC | | 2 | | 1 | | | | 3 | 1 | | 3 |
| Neo Heliopan^{®} OS | 2 | | | | | | | | 7 | | |
| Neo Heliopan^{®} E1000 | | 4 | | | | | | 5 | | | |
| Neo Heliopan^{®} AV | | 4 | 7.5 | 5 | | | | 5 | 4 | 7.5 | |
| Uvinul^{®} A PLUS | | | | | 1 | | 2 | | | | |
| Uvinul^{®} T 150 | 1 | | | | | | | | 1.3 | 1 | 1 |
| Tinosorb^{®} M | | 2 | | | 2 | | 2 | | | | |
| Tinosorb^{®} S | | 1 | | | 2 | | 2 | | | | |
| Parsol^{®} 1789 | 1 | | | | | | | | 2 | | 1 |
| Z-Cote^{®} HP 1 | 7 | 2 | 5 | | | 7 | 5 | | 6 | 2 | |
| Eusolex^{®} T 2000 | 5 | 2 | | | 10 | | | 10 | | 2 | |
| Veegum^{®} Ultra | 1.5 | | 1.5 | | | 1.5 | 1.2 | | 1 | | |
| Keltrol^{®} T | 0.5 | | 0.5 | | | 0.5 | 0.4 | | 0.5 | | |
| Cosmedia^{®} SP | | | 0.2 | 0.3 | | | 0.1 | | | 0.2 | |
| Pemulen^{®} TR 2 | | 0.3 | | 0.3 | | | | 0.2 | | | 0.3 |
| Ethanol | | 5 | | 8 | | | | | | | |
| Butylenglykol | 1 | | | 3 | 3 | | | | | 8 | 1 |
| Glycerin | 2 | 4 | 3 | 3 | | 3 | 3 | 3 | 5 | | 3 |
| Wasser/ Konservierungsmittel/ NaOH | ad 100/ q.s./ q.s. | | | | | | | | | | |

**Tabelle 5: O/W-Pflegeemulsionen**

| **Komponente** | **12** | **13** | **14** | **15** | **16** | **17** | **18** | **19** | **20** | **21** | **22** |
|---|---|---|---|---|---|---|---|---|---|---|---|
| **L = Lotion, C = Creme** | C | C | L | C | L | C | L | L | L | L | C |
| Eumulgin^{®} VL 75 | | | 5 | | 4 | | | | | | 2 |
| Generol^{®} R | | | | | | 2 | | | | | |
| Eumulgin^{®} B2 | | | | | | | | | | 1 | |
| Tween^{®} 60 | | | | | | | | | | 1 | |
| Cutina^{®} E 24 | | | | 0.5 | | | | | | | |
| Eumulgin^{®} SG | | | 0,1 | 0,5 | | 0,4 | | 0,2 | 0,1 | | |
| Lanette^{®} E | 0.5 | | | | | | | | | | |
| Amphisol^{®} K | 0,5 | 0.5 | | | | | | | | | |
| Natnumstearat | | | | | 1 | | | | | | |
| Emulgade^{®} PL 68/50 | | 2 | | 2 | | | | 3 | 4 | | |
| Tego^{®} Care 450 | | 1 | | | | | | | 1 | | |
| Cutina^{®} MD | 2 | 1 | 1 | 1 | | 5 | | | | 2 | |
| Lanette^{®} 14 | | | | | 1 | | | 2 | | 1 | |
| Lanette^{®} O | 2 | | | 2 | 1 | 3 | 1 | | 1 | 1 | 3 |
| Cutina^{®} PES | 1 | 2 | | 3 | 1 | | | | | | 3 |
| Novata^{®} AB | | | | | | | | | 1 | 1 | |
| Emery^{®} 1780 | | | | | | | | | | | 0.5 |
| Lanolin, wasserfrei, USP | | | | | | 4 | | | | | |
| Cosmedia^{®} DC | | | 2 | | | 1.5 | | | 1 | 1 | |
| Cetiol^{®} SB 45 | | | | | | | 2 | | | | |
| Cegesoft^{®} C 17 | 2 | | | | | | | | | | |
| **erfindungsgemäße Ölkomponente** | **5** | **5** | **4** | **4** | **3** | **4** | **5** | **4** | **5** | **10** | **2** |
| Myritol^{®} PC | 6 | | | | | 5 | | | | | |
| Myritor^{®} 331 | 2 | | 5 | | | | 2 | | | | 3 |
| Finsolv^{®} TN | | | | 3 | 5 | | | 3 | 3 | | 1 |
| Cetiol^{®} CC | | | | 3 | | | 4 | 3 | | | |
| Cetiol^{®} OE | | | | | 2 | | 2 | | 5 | | |
| Dow Corning DC^{®} 245 | | 2 | | | 1 | 4 | | | | 8 | 2 |
| Dow Corning DC^{®} 2502 | | 1 | | | 1 | | | | | | 3 |
| Prisorine^{®} 3758 | 3 | | | | | | | | | | 2 |
| Silikonöl Wacker AK^{®} 350 | | | | | 1 | | | | | | 1 |
| Cetiol^{®} 868 | | 2 | | | | | | | | | |
| Cetiol^{®} J 600 | | 2 | | 2 | | | | | | | |
| Ceraphyl^{®} 45 | | | | | | | 3 | | | | |
| Cetiol^{®} SN | | | | 5 | | | | | | | |
| Cetiol^{®} B | | | 5 | | | 5 | | 4 | | | 3 |
| Eutanol^{®} G | | 3 | 5 | | 5 | | | | | | |
| Cetiol^{®} PGL | | | | | | | | 5 | 2 | | |
| Dry Flo^{®} Plus | | 1 | | | | | | | | | 1 |
| SFE 839 | 1 | 1 | | | | | | | | | |
| Mandelöl | | | | | | 2 | | | | | |
| Photonyl^{®} LS | | | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum^{®} Ultra | | | | | | | | | 1 | | |
| Keltrol^{®} T | | | | | | | | | 0.5 | | |
| Cosmedia^{®} SP | 0.5 | | | | | 0.5 | 0.5 | 0.2 | | | 0.5 |
| Carbopol^{®} ETD 2001 | | 0.3 | | 0.3 | | | | | | | |
| Pemulen^{®} TR 2 | | | 0.3 | | | 0.3 | | | | | |
| Ethanol | | 5 | | 8 | | | | | | | 10 |
| Butylenglykol | 5 | | 2 | 3 | 3 | | | | | 8 | |
| Glycerin | 2 | 4 | 3 | 3 | | 7 | 5 | 3 | 5 | | |
| Wasser, Konservierungsmittel, NaOH | ad 100, q.s. (pH 6,5 - 7,5) | | | | | | | | | | |

### Formulierungen für den Sonnenschutz und die Hautpflege vom Typ Wasser in Öl

In Tabelle 6 werden Sonnenschutzformulierungen vom W/O Emulsionstyp, in Tabelle 7 werden Pflegeemulsionen beschrieben. Durch den Einsatz der erfindungsgemäßen Ölkomponente wird das sensorische Verhalten bei Applikation positiv beeinflusst. Die Mengenangaben beziehen sich jeweils auf Gew.-% der handelsüblichen Substanzen in der Gesamtzusammensetzung.

**Tabelle 6: W/O - Sonnenschutzformulierungen**

| **Ingredient** | **23** | **24** | **25** | **26** | **27** | **28** | **29** | **30** | **31** | **32** | **33** |
|---|---|---|---|---|---|---|---|---|---|---|---|
| **L = Lotion; C = Creme** | C | L | C | L | C | L | L | L | L | C | C |
| Dehymuls^{®} PGPH | 4 | 2 | 1 | 3 | 3 | 1 | 1 | 2 | 2 | 4 | 1 |
| Monomuls^{®} 90-018 | | | 2 | | | | | | | | |
| Lameform^{®} TGI | 2 | | 4 | | 3 | | | | | 1 | 3 |
| Abil^{®} EM 90 | | | | | | | 4 | | | | |
| Isolan^{®} PDI | | | | | | 4 | | 2 | | | |
| Zinkstearat | 1 | | | 1 | 1 | | | 1 | | 1 | |
| Bienenwachs | 1 | | 5 | 1 | | | | 5 | | 7 | 5 |
| Tego^{®} Care CG | | | | | 1 | | | | | | 0.5 |
| Prisorine^{®} 3505 | | | 1 | | | 1 | 1 | | | | 1 |
| Cosmedia DC | 3 | 4 | 2 | 1 | 1 | 2 | 2 | 2 | 3 | 1 | 1 |
| erfindungsgemäße Ölkomponente | 5 | 4 | 4 | 3 | 2 | 4 | 3 | 4 | 2 | 3 | 5 |
| Myritol^{®} 331 | 2 | | | | 3 | 6 | | | | | 3 |
| Finsolv^{®} TN | | | | 5 | | | 2 | | | | |
| Cetiol^{®} CC | 5 | | 2 | | 4 | 2 | | | 2 | 3 | 5 |
| Tegosoft DEC | | 4 | | 3 | | | 5 | 5 | | | |
| Cetiol^{®} OE | | | | | 4 | | 5 | | 4 | 2 | |
| Dow Corning^{®} DC 244 | | | 3 | | | | 2 | | 2 | 4 | |
| Dow Corning^{®} DC 2502 | 1 | | 1 | | 2 | 1 | | | | | 1 |
| Silikonöl Wacker AK 350 | | 1 | | 4 | | | | 3 | | | |
| Cetiol^{®} PGL | | 3 | | | | 2 | | | 4 | | |
| Cophero^{®} F 1300 | 1 | | | | | | | | | | |
| Magnesium sulfat x 7 H₂O | 1 | | | | | | | | | | |
| Neo Heliopan^{®} Hydro (Na-Salz) | 2 | | 22 | | 3 | 3 | | | 1 | | 2 |
| Neo Heliopan^{®} 303 | | 5 | | | | | | | 4 | | 4 |
| Uvasorb^{®} HEB | 1 | | | 1 | 1 | | | | | | 2 |
| Neo Heliopan^{®} MBC | 2 | | | | | 2 | 2 | 2 | | | |
| Uvinul^{®} A plus | | | | | 2 | | | | 3 | 3 | |
| Neo Heliopan^{®} AP (Na-Salz) | | 2 | 2 | | 1 | | | | 1 | | 6 |
| Neo Heliopan^{®} AV | 3 | | 4 | 6 | 4 | 7.5 | 4 | 5 | | | 1 |
| Uvinul^{®} T 150 | 1 | 1 | | | 2.5 | | | 1 | | | |
| Parsol^{®} 1789 | 2 | 1 | | | | | 2 | | 2 | 2 | |
| Zinkoxid NDM | | | | | | 10 | | 3 | | | 4 |
| Tinosorb^{®} M | | 3 | | 3 | | | | 2 | | 2 | |
| Tinosorb^{®} S | | 3 | | 3 | | | | 2 | | 2 | |
| Eusolex^{®} T Aqua | | | 8 | | | | | 5 | | | |
| Eusolex^{®} T 2000 | | | | | 5 | | 3 | 3 | | | 4 |
| Ethanol | | | | | | | | | | 8 | |
| Glycerin | 5 | 3 | 3 | 3 | 5 | 3 | 2 | 3 | 10 | 4 | 3 |
| Wasser, Konservierungsmittel | ad 100, q.s. | | | | | | | | | | |

**Tabelle 7: W/O-Pflegeemulsionen**

| **Komponente** | **34** | **35** | **36** | **37** | **38** | **39** | **40** | **41** | **42** | **43** | **44** |
|---|---|---|---|---|---|---|---|---|---|---|---|
| L = Lotion. C = Creme | C | L | C | L | C | L | L | L | C | C | C |
| Dehymuls^{®} PGPH | 1 | 3 | 1 | 2 | 3 | 1 | 1 | 2 | 1 | 1 | 1 |
| Monomuls^{®} 90-018 | 2 | | | | | | | | 2 | | 2 |
| Lameform^{®} TGI | 4 | 1 | | | 3 | | | 1 | 4 | 3 | 3 |
| Abil^{®} EM 90 | | | | | | | 4 | | | | |
| Isolan^{®} PDI | | | | | | 4 | | | | | |
| Glucate^{®} DO | | | | 5 | | | | | | | |
| Arlacel^{®} 83 | | | 5 | | | | | | | | |
| Dehymuls^{®} FCE | | | | | | | | | | | |
| Dehymuls^{®} HRE 7 | | | | | | | | 4 | | 1 | |
| Zinkstearat | 2 | 1 | | 1 | 1 | | | 1 | 1 | 1 | |
| Mikrokristallines Wachs | | | 5 | | | 2 | | | | | 5 |
| Bienenwachs | 4 | | | 1 | | | | 1 | 4 | 7 | |
| Tego Gare^{®} CG | | | | | 1 | | | | | | 0.5 |
| Prisorine^{®} 3505 | | | 1 | 1 | | 1 | 1 | | | | 1 |
| Dry Flo^{®} Plus | | | | | | | | | | | |
| SFE 839 | | | | | | | 3 | | | | |
| Emery^{®} 1780 | 1 | | | | | | | | | | 1 |
| Lanolin: anhvdrous USP | | | 5 | | | | | | | 4 | |
| **erfindungsgemäße Ölkomponente** | **3** | **4** | **2** | **12** | **10** | **2** | **2** | **6** | **3** | **12** | **1** |
| Cegesoft^{®} C 17 | | | 3 | | | | | | | 1 | |
| Myritol^{®} PC | | | | | | 2 | | 4 | | | |
| Myritol^{®} 331 | 6 | | | | 2 | 6 | 2 | | | | 8 |
| Finsolv^{®} TN | | | | 5 | | 2 | 5 | | | | |
| Cetiol^{®} A | | 6 | | | | 4 | | | | | |
| Cetiol^{®} CC | | 8 | | | 2 | 2 | 2 | | | | 5 |
| Cetiol^{®} SN | | 5 | | | | | | 3 | | | |
| Cetiol^{®} OE | 3 | | | | 4 | | 2 | | 4 | 2 | |
| Dow Coming DC^{®} 244 | | | | | 1 | | 2 | | | | |
| Dow Coming DC^{®} 2502 | | | 1 | | 2 | | | | | | |
| Prisonne^{®} 3758 | | | | | 3 | | | | | | |
| Silikonöl Wacker AK^{®} 350 | | | | 4 | | | | 3 | | | |
| Cetiol^{®} 868 | | | | | | | | | | 2 | 7 |
| Cetiol^{®} J 600 | | | 4 | | | 2 | | | | | |
| Ceraphyl^{®} 45 | | | | 2 | | | | 2 | 2 | 6 | |
| Mineralöl | | | | | 4 | | | | | | |
| Cetiol^{®} B | | | 2 | 4 | | | | | | 3 | |
| Eutanol^{®} G 16 | | 1 | | | | | | | | 3 | |
| Eutanor^{®} G | | | 3 | | | | | 8 | | | |
| Cetiol^{®} PGL | | | | | | 4 | | | 9 | | |
| Mandelöl | | | | | 1 | | 5 | | | | |
| Insect Repellent^{®} 3535 | 2 | | | | | | | | | | |
| N,N-Diethyl-m-toluamid | | | | 3 | | - | | 5 | | | |
| Photonyl^{®} LS | 2 | 2 | | | | | | | | | |
| Panthenol | 1.0 | | | | | | | | | | |
| Bisabolol | 0.2 | | | | | | | | | | |
| Tocopherol / Tocopheryl Acetate | 1.0 | | | | | | | | | | |
| Maqnesiumsulfat x 7 Wasser | 1 | | | | | | | | | | |
| Bentone^{®} 38 | | | | | 1 | | | | | | |
| Propylencarbonat | | | | | 0.5 | | | | | | |
| Ethanol | | | | | | | | | | 8 | |
| Butylene Glycol | | | 2 | 6 | | | 2 | 5 | | | 2 |
| Glycerin | 5 | 3 | 3 | | 5 | 3 | 12 | | 10 | 4 | |
| Wasser, Konservierungsmittel | Ad 100, q.S. | | | | | | | | | | |

### Anhang

| | | | |
|---|---|---|---|
| 1) | Abil^{®} EM 90 | 8) | Bentone^{®} 38 |
| | INCI: Cetyl Dimethicone Copolyol | | INCI: Quatemium-18 Hectorite |
| | Hersteller: Tego Cosmetics (Goldschmidt) | | Hersteller: Rheox (Elementis Specialties) |
| | | | |
| 2) | Allianz^{®} OPT | 9) | Carbopol^{®} 980 |
| | INCI: Acrylates/C12-22 Alkyl Methacrylate Copolymer | | INCI: Carbomer |
| | | | Hersteller: Goodrich |
| | Hersteller: Rohm und Haas | | |
| | | 10) | Carbopol^{®} 2984 |
| 3) | Amphisol^{®} K | | INCI: Carbomer |
| | INCI: Potassium Cetyl Phosphate | | Hersteller: Noveon, Inc. |
| | Hersteller: Hoffmann La Roche | | |
| | | 11) | Carbopol^{®} ETD 2001 |
| 4) | Antaron^{®} V 220 | | INCI: Carbomer |
| | INCI: PVP/Eicosene Copolymer | | Hersteller: Noveon, Inc. |
| | Hersteller: GAF General Aniline Firm | | |
| | Corp. (IPS-Global) | 12) | Carbopol^{®} Ultrez 10 |
| | | | INCI: Carbomer |
| 5) | Antaron^{®} V 216 | | Hersteller: Noveon, Inc. |
| | INCI: PVP/Hexadecene Copolymer | | |
| | Hersteller: GAF General Aniline Firm Corp. (IPS-Global) | 13) | Cegesoft^{®} C 17 |
| | | | INCI: Myristyl Lactate |
| | | | Hersteller: Cognis Deutschland GmbH, Grünau |
| 6) | Ariacel^{®} 83 | | |
| | INCI: Sorbitan Sesquioleate | | |
| | Hersteller: Uniqema (ICI Surfacants) | 14) | Cegesoft^{®} PFO |
| | | | INCI: Passiflora Incamata (EU) |
| 7) | Arlacel^{®} P135 | | Hersteller: Cognis Deutschland GmbH |
| | INCI: PEG-30 Dipolyhydroxystearate | | |
| | Hersteller: Uniqema (ICI Surfacants) | | |
| 15) | Cegesoft^{®} PS 6 | 23) | Cetiol^{®} OE |
| | INCI: Olus | | INCI: Dicaprylyl Ether |
| | Hersteller: Cognis Deutschland GmbH | | Hersteller: Cognis Deutschland GmbH |
| | | | |
| 16) | Ceraphyl^{®} 45 | 24) | Cetiol^{®} PGL |
| | INCI: Diethylhexyl Malate | | INCI: Hexyldecanol, Hexyldecyl Laurate |
| | Hersteller: International Specialty Products | | Hersteller: Cognis Deutschland GmbH |
| | | | 25) Cetiol^{®} S |
| 17) | Cetiol^{®} 868 | | INCI: Diethylhexylcyclohexane |
| | INCI: Ethylhexyl Stearate | | Hersteller: Cognis Deutschland GmbH |
| | Hersteller: Cognis Deutschland GmbH | | |
| | | 26) | Cetiol^{®} SB 45 |
| 18) | Cetiol^{®}A | | INCI: Shea Butter Butyrospermum Parkii (Linne) |
| | INCI: Hexyl Laurate | | |
| | Hersteller: Cognis Deutschland GmbH | | Hersteller: Cognis Deutschland GmbH |
| | | | |
| 19) | Cetiol^{®}B | 27) | Cetiol^{®} SN |
| | INCI: Dibutyl Adipate | | INCI: Cetearyl Isononanoate |
| | Hersteller: Cognis Deutschland GmbH | | Hersteller: Cognis Deutschland GmbH |
| | | | |
| 20) | Cetiol^{®} CC | 28) | Copherol^{®} F 1300 C |
| | INCI: Dicaprylyl Carbonate | | INCI: Tocopherol |
| | Hersteller: Cognis Deutschland GmbH | | Hersteller Cognis Deutschland GmbH |
| | | | |
| 21) | Cetiol^{®} J600 | 29) | Copherol 1250 C |
| | INCI: Oleyl Erucate | | INCI: Tocopheryl Acetate |
| | Hersteller: Cognis Deutschland GmbH | | Hersteller: Cognis Deutschland GmbH |
| | | | |
| 22) | Cetiol^{®} LC | 30) | Cosmedia^{®} DC |
| | INCI: Coco-Caprylate/Caprate | | INCI: Hydrogenated Dimer Dilinoleyl / Dimethylcarbonate Copolymer |
| | Hersteller: Cognis Deutschland GmbH | | |
| | Hersteller: Cognis Deutschland GmbH | 38) | Dehymuls^{®} PGPH |
| | | | INCI: Polyglyceryl-2 Dipolyhydroxystearate |
| 31) | Cosmedia^{®} SP | | Hersteller: Cognis Deutschland GmbH |
| | INCI: Sodium Polyacrylate | | |
| | Hersteller: Cognis Deutschland GmbH | 39) | Dow Corning^{®} 244 Fluid |
| | | | INCI: Cyclomethicone |
| 32) | Cutina^{®} E 24 | | Hersteller: Dow Corning |
| | INCI: PEG-20 Glyceryl Stearate | | |
| | Hersteller: Cognis Deutschland GmbH | 40) | Dow Corning^{®}246 Fluid |
| | | | INCI: Cyclopentasiloxane |
| 33) | Cutina^{®} HR | | Hersteller: Dow Coming |
| | INCI: Hydrogenated Castor Oil | | |
| | Hersteller: Cognis Deutschland GmbH | 41) | Dow Corning^{®} 2502 |
| | | | INCI: Cetyl Dimethicone |
| 34) | Cutina^{®} MD | | Hersteller: Dow Coming |
| | INCI: Glyceryl Stearate | | |
| | Hersteller: Cognis Deutschland GmbH | 42) | Dry^{®}Flo Plus |
| | | | INCI: Aluminium Starch Octenylsuccinate |
| 35) | Cuitina^{®} PES | | Hersteller: National Starch |
| | INCI: Pentaerythrityl Distearate | | |
| | Hersteller: Cognis Deutschland GmbH | 43) | Elfacos^{®}ST 37 |
| | | | INCI: PEG-22 Dodecyl Glycol Copolymer |
| 36) | Dehymuls^{®} FCE | | Hersteller: Akzo-Nobel |
| | INCI: Dicocoyl Pentaerythrityl Distearyl Citrate | | |
| | | 44) | Elfacos^{®}ST 9 |
| | Hersteller: Cognis Deutschland GmbH | | INCI: PEG-45 Dodecyl Glycol Copolymer |
| | | | Hersteller: Akzo-Nobel |
| 37) | Dehymuls^{®} HRE 7 | | |
| | INCI: PEG-7 Hydrogenated Castor Oil | 45) | Emery^{®} 1780 |
| | Hersteller: Cognis Deutschland GmbH | | INCI: Lanolin Alcohol |
| | | | Hersteller: Cognis Corporation (Emery) |
| 46) | Emulgade^{®} CM | 52) | Eumulgin^{®} SG |
| | INCI: Cetearyl Isononanoate and Ceteareth-20 and Cetearyl Alcohol and Glyceryl Stearate and Glycerin and Ceteareth-12 and Cetyl Palmitate | | INCI:Sodium Stearoyl Glutamate |
| | | | Hersteller: Cognis Deutschland GmbH |
| | | 53) | Eumulgin^{®} VL 75 |
| | | | INCI: Lauryl Glucoside (and) Polyglyceryl-2 Dipolyhydroxystearate (and) Glycerin |
| | Hersteller: Cognis Deutschland GmbH | | |
| | | | Hersteller: Cognis Deutschland GmbH |
| 47) | Emulgade^{®}PL 68/50 | | |
| | INCI: Cetearyl Glucoside, Cetearyl Alcohol | 54) | Eusolex^{®} OCR |
| | | | INCI: Octocrylene |
| | Hersteller: Cognis Deutschland GmbH | | Hersteller: Merck |
| | | | |
| 48) | Emulgade^{®} SE - PF | 55) | Eusolex^{®} T 2000 |
| | INCI: Glyceryl Stearate (and) Ceteareth-20 (and) Ceteareth-12 (and) Cetearyl Alcohol (and) Cetyl Palmitate | | INCI: Titanium Dioxide, Alumina, Simethicone |
| | | | Hersteller: Merck |
| | Hersteller: Cognis Deutschland GmbH | | |
| | | | 56) Eusolex^{®} T AQUA |
| 49) | Eumulgin^{®} B1 | | INCI: Water and Titanium Dioxide and Alumina and Sodium Metaphosphate and Phenoxyethanol and Sodium Methylparaben |
| | INCI: Ceteareth-12 | | |
| | Hersteller: Cognis Deutschland G mbH | | |
| | | | |
| 50) | Eumulgin^{®} B 2 | | Hersteller: Merck |
| | INCI: Ceteareth- 20 | | |
| | Hersteller: Cognis Deutschland GmbH | 57) | Eutanol^{®} G |
| | | | INCI: Octyldodecanol |
| 51) | Eumulgin^{®} HRE 40 | | Hersteller: Cognis Deutschland GmbH |
| | INCI: PEG-40 Hydrogenated Castor Oil | | |
| | Hersteller: Cognis Deutschland GmbH | 58) | Eutanol^{®}G 16 |
| | | | INCI: Hexyldecanol |
| | | | Hersteller: Cognis Deutschland GmbH |
| 59) | Eutanol^{®}G 16 S | 67) | Insect Repellent^{®} 3535 |
| | INCI: Hexyldecyl Stearate | | INCI: Ethyl Butylacetylaminopropionate |
| | Hersteller: Cognis Deutschland GmbH | | Hersteller : EMD Chemicals Inc |
| | | | |
| 60) | Finsolv^{®} TN | 68) | Isolan^{®} PDI |
| | INCI: C 12/15 Alkyl Benzoate | | INCI: Diisostearoyl Polyglyceryl-3 Diisos-tearate |
| | Hersteller: Findex (Nordmann/Rassmann) | | |
| | | | Hersteller: Goldschmidt AG |
| 61) | Generol^{®} R | | |
| | INCI: Brassica Campestris (Rapseed) Sterols | 69) | Kelfrol^{®} T |
| | | | INCI: Xanthan Gum |
| | Hersteller: Cognis Deutschland GmbH | | Hersteller: CP Kelco |
| | | | |
| 62) | Glucate^{®} DO | 70) | Lameform^{®} TGI |
| | INCI: Methyl Glucose Dioleate | | INCI: Polyglyceryl-3 Diisostearate |
| | Hersteller: NRC Nordmann/Rassmann | | Hersteller: Cognis Deutschland GmbH |
| | | | |
| 63) | Hispagel^{®} 200 | 71) | Lanette^{®} 14 |
| | INCI: Glycerin, Glyceryl Polyacrylate | | INCI: Myristyl Alcohol |
| | Hersteller: Cognis Deutschland GmbH | | Hersteller: Cognis Deutschland GmbH |
| | | | |
| 64) | Hostaphat^{®} KL 340 N | 72) | Lanette 18 |
| | INCI: Trilaureth-4 Phosphate | | INCI: Stearyl Alcohol |
| | Hersteller: Clariant | | Hersteller: Cognis Deutschland GmbH |
| | | | |
| 65) | Hydagen^{®} C.A.T. | 73) | Lanette^{®} 22 |
| | INCI Triethyl Citrate | | INCI: Behenyl Alcohol |
| | Hersteller: Cognis Deutschland GmbH | | Hersteller: Cognis Deutschland GmbH |
| | | | |
| | 66) Hydagen^{®} DCMF | 74) | Lanette^{®} E |
| | INCI : Chitosan | | INCI: Sodium Cetearyl Sulfate |
| | Hersteller: Cognis Deutschland GmbH | | Hersteller: Cognis Deutschland GmbH |
| 75) | Lanette^{®} O | 83) | Neo Heliopan^{®} 303 |
| | INCI: Cetearyl Alcohol | | INCI: Octocrylene |
| | Hersteller: Cognis Deutschland GmbH | | Hersteller: Symrise |
| | | | |
| 76) | Locron^{®} L | 84) | Neo Heliopan^{®} AP |
| | INCI: Aluminium Chlorhydrate | | INCI: Disodium Phenyl Dibenzimidazole Tetrasulfonate |
| | Hersteller: Clariant | | |
| | | | Hersteller: Symrise |
| 77) | Lucentite^{®} SAN | | |
| | INCI: Quatemium-18 Hectoritr | 85) | Neo Heliopa^{®} AV |
| | Hersteller: Co-Op Chemical Co., Ltd. | | INCI: Ethylhexyl Methoxycinnamate |
| | | | Hersteller: Symrise |
| 78) | Monomuls^{®} 90-O18 | | |
| | INCI: Glyceryl Oleate | 86) | Neo Heliopan^{®} BB |
| | Hersteller: Cognis Deutschland GmbH | | INCI: Benzophenone-3 |
| | | | Hersteller: Symrise |
| 79) | Myrj^{®} 51 | | |
| | INCI: PEG-30-Sterate | 87) | Neo Heliopan^{®} E 1000 |
| | Hersteller: Uniqema | | INCI: Isoamyl-p-Methoxycinnamate |
| | | | Hersteller: Symrise |
| 80) | Myritol^{®} 312 | | |
| | INCI: Caprylic/Capric Triglyceride | 81) | Neo Heliopan^{®} Hydro |
| | Hersteller: Cognis Deutschland GmbH | | INCI: Phenylbenzimidazole Sulfonic Acid |
| | | | Hersteller: Symrise |
| 81) | Myritol^{®} 331 | | |
| | INCI: Cocoglycerides | 82) | Neo Heliopan^{®} MBC |
| | Hersteller: Cognis Deutschland GmbH | | INCI: 4-Methylbenzylidene Camphor |
| | | | Hersteller: Symrise |
| 82) | Myritol^{®} PC | | |
| | INCI: Propylene Glycol | 83) | Neo Heliopan^{®} OS |
| | Dicaprylate/Dicaprate | | INCI: Ethylhexyl Salicylate |
| | Hersteller: Cognis Deutschland GmbH | | Hersteller: Symrise |
| 84) | Novata^{®} AB | | Hersteller: Reheis, Inc |
| | INCI: Cocoglycerides | | |
| | Hersteller: Cognis Deutschland GmbH | 91) | SFE^{®} 839 |
| | | | INCI: Cyclopentasiloxane and Dimethi-cone/Vinyl Dimethicone Crosspolymer |
| 85) | Parsol^{®} 1789 | | |
| | INCI: Butyl Methoxydibenzoylmethane | | Hersteller: GE Silicones |
| | Hersteller: Hoffmann-La Roche (Givaudan) | | |
| | | 92) | Silikonöl Wacker AK^{®} 350 |
| | | | INCI: Dimethicone |
| 86) | Pemulen^{®} TR-2 Polymer | | Hersteller: Wacker |
| | INCI: Acrylates / C10-30 Alkylacrylate Crosspolymer | | |
| | | 93) | Tego^{®} Care 450 |
| | Hersteller: Noveon, Inc. | | INCI: Polyglyceryl-3 Methylglucose Distearate |
| | | | |
| 87) | Photonyl^{®} LS | | Hersteller: Tego Cosmetics (Goldschmidt) |
| | INCI: Arginine, Disodium Adenosine Triphosphate, Mannitol, Pyridoxine HCL, Phenylalanine, Tyrosine | | |
| | | 94) | Tego^{®} Care CG 90 |
| | | | INCI: Cetearyl Glucoside |
| | Hersteller: Laboratoires Serobiologiques (Cognis) | | Hersteller: Goldschmidt |
| | | | |
| | | 95) | Tegosoft^{®} DEC |
| 88) | Prisorine^{®} 3505 | | INCI: Diethylhexyl Carbonate |
| | INCI: Isostearic Acid | | Hersteller: Goldschmidt |
| | Hersteller: Uniqema | | |
| | | 96) | Tinosorb^{®} S |
| 89) | Prisorine^{®} 3758 | | INCI: Bis-Ethylhexyloxyphenol Metho-xyphenyl Triazine |
| | INCI: Hydrogenated Polyisobutene | | |
| | Hersteller: Uniqema | | Hersteller: Ciba Specialty Chemicals Corporation |
| | | | |
| 90) | Rezal 36G | | |
| | INCI: Aluminum Zirconium Tetrachlorohydrex GLY | | |
| 97) | Tinosorb^{®} M | 104) | Veegum^{®} Plus |
| | INCI: Methylene Bis-Benzotriazolyl Tetramethylbutylphenol | | INCI: Magnesium Aluminum Silicate and Cellulose Gum |
| | | | |
| | Herstelller: Ciba Specialty Chemicals Corporation | | Hersteller: R. T. Vanderbilt Company, Inc |
| | | | |
| | | 105) | Z-Cote^{®} HP 1 |
| 98) | Tween^{®} 60 | | INCI: Zinc Oxide and Triethoxycaprylylsilane |
| | INCI: Polysorbate 60 | | Hersteller: BASF |
| | Hersteller: Uniqema (ICI Surfactants) | | |
| | | 106) | Zinc Oxide NDM |
| 99) | Uvasorb^{®} HEB | | INCI: Zinc Oxide |
| | INCI: Diethylhexyl Butamido Triazone | | Hersteler: Symrise |
| | Hersteller: 3V Inc. | | |
| | | | |
| 100) | Unirep^{®} U-18 | | |
| | INCI: Dimethyl Phthalate and Diethyl Toluamide and Ethyl Hexanediol | | |
| | | | |
| | Hersteller: Induchem AG | | |
| | | | |
| 101) | Uvinul^{®} T 150 | | |
| | INCI: Ethylhexyl Triazone | | |
| | Hersteller: BASF | | |
| | | | |
| 102) | Uvinul^{®} A plus | | |
| | INCI: Diethylamino Hydroxybenzoyl Hexyl Benzoate | | |
| | | | |
| | Hersteller: BASF | | |
| | | | |
| 103) | Veegum^{®} Ultra | | |
| | INCI: Magnesium Aluminium Silicate | | |
| | Hersteller: R. T. Vanderbilt Company, Inc | | |

## Patentansprüche

1. Fettsäureestergemisch von 2-Ethylhexanol und Fettsäuren, wobei die Summe der C12- und C14- 2-Ethylhexylfettsäureester größer gleich 85%, bezogen auf die Gesamtsumme der Fettsäureester, beträgt.

2. Fettsäureestergemisch nach Anspruch 1, **dadurch gekennzeichnet, dass** die Summe der C12- und C14- 2-Ethylhexylfettsäureester größer gleich 90%, insbesondere größer gleich 95%, bezogen auf die Gesamtsumme der Fettsäureester, beträgt.

3. Fettsäureestergemisch von 2-Ethylhexanol und Fettsäuren nach einem der vorgenannten Ansprüche wobei die Summe der 2-Ethylhexylfettsäureester mit einer C-Kettenlänge von kleiner gleich 10 kleiner gleich 3%, bezogen auf die Gesamtsumme der Fettsäureester, beträgt.

4. Fettsäureestergemisch nach Anspruch 3, **dadurch gekennzeichnet, dass** die Summe der 2-Ethylhexylfettsäureester mit einer C-Kettenlänge von kleiner gleich 10, kleiner gleich 2 %, insbesondere kleiner gleich 1,5 % bezogen auf die Gesamtsumme der Fettsäureester, beträgt.

5. Fettsäureestergemisch von 2-Ethylhexanol und Fettsäuren nach einem der vorgenannten Ansprüche wobei die Summe der 2-Ethylhexylfettsäureester mit einer C-Kettenlänge von größer gleich 16, kleiner gleich 8%, bezogen auf die Gesamtsumme der Fettsäureester, beträgt.

6. Fettsäureestergemisch von 2-Ethylhexanol und Fettsäuren nach Anspruch 5, wobei die Summe der 2-Ethylhexylfettsäureester mit einer C-Kettenlänge von größer gleich 16, kleiner gleich 8%, insbesondere kleiner gleich 4 %, bevorzugt kleiner gleich 3%, insbesondere kleiner gleich 2% beträgt.

7. Fettsäureestergemisch von 2-Ethylhexanol und Fettsäuren nach einem der vorgenannten Ansprüche wobei die 2-Ethylhexylfettsäureester folgende C-Kettenvertellung aufweisen
(a) C-12 größer gleich 60%, bevorzugt größer gleich 65%, insbesondere größer gleich 70 % und
(b) C-14 zwischen 15% und 40%, insbesondere zwischen 20% und 35%, bevorzugt zwischen 25% und 30%.
bezogen auf die Gesamtsumme der Fettsäureester.

8. Fettsäureestergemisch nach Anspruch 7, **dadurch gekennzeichnet, dass** die Summe der 2-Ethylhexylfettsäureester mit einer C-Kettenlänge von kleiner gleich 10, kleiner gleich 3%, insbesondere kleiner gleich 2%, bevorzugt kleiner gleich 1%, bezogen auf die Gesamtsumme der Fettsäureester, beträgt.

9. Fettsäureestergemisch nach Anspruch 7 und/oder 8, **dadurch gekennzeichnet, dass** Summe der 2-Ethylhexylfettsäureester mit einer C-Kettenlänge von größer gleich 16, kleiner gleich 8%, insbesondere kleiner gleich 4%, insbesondere kleiner gleich 3%, bevorzugt kleiner gleich 2% , bezogen auf die Gesamtsumme der Fettsäureester, beträgt.

10. Verwendung der Fettsäureestergemische nach einem der vorgenannten Ansprüche in kosmetischen und/oder pharmazeutischen Zubereitungen.

11. Verwendung nach Anspruch 10 als Ölkomponente.

## Claims

1. Fatty acid ester mixture of 2-ethylhexanol and fatty acids, where the sum of the C12 and C14 2-ethylhexyl fatty acid esters is greater than or equal to 85%, based on the total sum of the fatty acid esters.

2. Fatty acid ester mixture according to Claim 1, **characterized in that** the sum of the C12 and C14 2-ethylhexyl fatty acid esters is greater than or equal to 90%, in particular greater than or equal to 95%, based on the total sum of the fatty acid esters.

3. Fatty acid ester mixture of 2-ethylhexanol and fatty acids according to one of the preceding claims, where the sum of the 2-ethylhexyl fatty acid esters with a carbon chain length of less than or equal to 10 is less than or equal to 3%, based on the total sum of the fatty acid esters.

4. Fatty acid ester mixture according to Claim 3, **characterized in that** the sum of the 2-ethylhexyl fatty acid esters with a carbon chain length of less than or equal to 10, is less than or equal to 2%, in particular less than or equal to 1.5%, based on the total sum of the fatty acid esters.

5. Fatty acid ester mixture of 2-ethylhexanol and fatty acids according to one of the preceding claims, where the sum of the 2-ethylhexyl fatty acid esters with a carbon chain length of greater than or equal to 16 is less than or equal to 8%, based on the total sum of the fatty acid esters.

6. Fatty acid ester mixture of 2-ethylhexanol and fatty acids according to Claim 5, where the sum of the 2-ethylhexyl fatty acid esters with a carbon chain length of greater than or equal to 16 is greater than or equal to 8%, in particular less than or equal to 4%, preferably less than or equal to 3%, in particular less than or equal to 2%.

7. Fatty acid ester mixture of 2-ethylhexanol and fatty acids according to one of the preceding claims, where the 2-ethylhexyl fatty acid esters have the following carbon chain distribution
(a) C-12 greater than or equal to 60%, preferably greater than or equal to 65%, in particular greater than or equal to 70% and
(b) C-14 between 15% and 40%, in particular between 20% and 35%, preferably between 25% and 30%,
based on the total sum of the fatty acid esters.

8. Fatty acid ester mixture according to Claim 7, **characterized in that** the sum of the 2-ethylhexyl fatty acid esters with a carbon chain length of less than or equal to 10 is less than or equal to 3%, in particular less than or equal to 2%, preferably less than or equal to 1%, based on the total sum of the fatty acid esters.

9. Fatty acid ester mixture according to Claim 7 and/or 8, **characterized in that** the sum of the 2-ethylhexyl fatty acid esters with a carbon chain length of greater than or equal to 16 is less than or equal to 8%, in particular less than or equal to 4%, in particular less than or equal to 3%, preferably less than or equal to 2%, based on the total sum of the fatty acid esters.

10. Use of the fatty acid ester mixtures according to one of the preceding claims in cosmetic and/or pharmaceutical preparations.

11. Use according to Claim 10 as oil component.

## Revendications

1. Mélange d'esters d'acides gras de 2-éthylhexanol et d'acides gras, la somme des esters 2-éthylhexyliques d'acides gras en C₁₂ et en C₁₄ étant supérieure à 85%, par rapport à la somme totale des esters d'acides gras.

2. Mélange d'esters d'acides gras selon la revendication 1, **caractérisé en ce que** la somme des esters 2-éthylhexyliques d' acides gras en C₁₂ et en C₁₄ est supérieure à 90%, en particulier supérieure à 95%, par rapport à la somme totale des esters d'acides gras.

3. Mélange d'esters d'acides gras de 2-éthylhexanol et d'acides gras selon l'une quelconque des revendications susmentionnées, la somme des esters 2-éthylhexyliques d'acides gras présentant une longueur de chaîne carbonée inférieure à 10 étant inférieure à 3%, par rapport à la somme totale des esters d'acides gras.

4. Mélange d'esters d'acides gras selon la revendication 3, **caractérisé en ce que** la somme des esters 2-éthylhexyliques d'acides gras présentant une longueur de chaîne carbonée inférieure à 10 est inférieure à 2%, en particulier inférieure à 1,5%, par rapport à la somme totale des esters d'acides gras.

5. Mélange d'esters d'acides gras de 2-éthylhexanol et d'acides gras selon l'une quelconque des revendications susmentionnées, la somme des esters 2-éthylhexyliques d'acides gras présentant une longueur de chaîne carbonée supérieure à 16 étant inférieure à 8%, par rapport à la somme totale des esters d'acides gras.

6. Mélange d'esters d'acides gras de 2-éthylhexanol et d'acides gras selon la revendication 5, la somme des esters 2-éthylhexyliques d'acides gras présentant une longueur de chaîne carbonée supérieure à 16 étant inférieure à 8%, en particulier inférieure à 4%, de préférence inférieure à 3%, en particulier inférieure à 2%.

7. Mélange d'esters d'acides gras de 2-éthylhexanol et d'acides gras selon l'une quelconque des revendications susmentionnées, les esters 2-éthylhexyliques d'acides gras présentant la répartition suivante des chaînes carbonées
(a) C-12 supérieur ou égal à 60%, de préférence supérieur ou égal à 65%, en particulier supérieur ou égal à 70% et
(b) C-14 entre 15% et 40%, en particulier entre 20% et 35%, de préférence entre 25% et 30%,
par rapport à la somme totale des esters d'acides gras.

8. Mélange d'esters d'acides gras selon la revendication 7, **caractérisé en ce que** la somme des esters 2-éthylhexyliques d'acides gras présentant une longueur de chaîne carbonée inférieure à 10 est inférieure à 3%, en particulier inférieure à 2%, de préférence inférieure à 1%, par rapport à la somme totale des esters d'acides gras.

9. Mélange d'esters d'acides gras selon la revendication 7 et/ou 8, **caractérisé en ce que** la somme des esters 2-éthylhexyliques d'acides gras présentant une longueur de chaîne carbonée supérieure à 16 est inférieure à 8%, en particulier inférieure à 4%, en particulier inférieure à 3%, de préférence inférieure à 2%, par rapport à la somme totale des esters d'acides gras.

10. Utilisation des mélanges d'esters d'acides gras selon l'une quelconque des revendications susmentionnées dans des compositions cosmétiques et/ou pharmaceutiques.

11. Utilisation selon la revendication 10 comme composant huileux.
